# EUROPEAN PATENT SPECIFICATION

(11) **EP 1 994 950 B1**
(45) Date of publication and mention of the grant of the patent: **20.02.2013**
(21) Application number: 08156469.2
(22) Date of filing: 19.05.2008
(51) Int. Cl.: A61L 31/04, A61L 31/10, A61L 31/14, A61L 31/16

(54) **A composition for the release of active principles from implant devices**
Zusammensetzung zur Freigabe von aktiven Prinzipien aus Implantatvorrichtungen
Composition pour la libération de principes actifs de dispositifs d'implant

(30) Priority: 21.05.2007 IT TO20070356
(43) Date of publication of application: 26.11.2008
(73) Proprietor: CID S.p.A., 13040 Saluggia (VC) (IT)
(72) Inventor: Grignani, Andrea, 10023, Chieri (Torino) (IT); Zambaldi, Ilaria, 10015, Ivrea (Torino) (IT); Strasly, Marina, 10020, Baldissero Torinese (Torino) (IT); Curcio, Maria, 13040, Saluggia (Vercelli) (IT)
(74) Representative: Freyria Fava, Cristina

(56) References cited:
- WO-A-2008/063576
- US-A1- 2006 198 867
- US-A1- 2007 026 035
- ANTONIO L. BARTORELLI ET AL: 'Synergy of Passive Coating and Targeted Drug Delivery:. The Tacrolimus-Eluting Janus CarboStent' JOURNAL OF INTERVENTIONAL CARDIOLOGY vol. 16, no. 6, 01 December 2003, pages 499 - 505, XP055030093 ISSN: 0896-4327

## Description

### FIELD OF THE INVENTION

The present invention relates to compositions for the release of active principles from implant devices such as e.g. implantable prostheses.

### DESCRIPTION OF THE RELATED ART

Associating active principles to implant devices such as implantable prostheses is an established technique. So-called Drug Eluting Stents (DESs) i.e. stents carrying pharmaceutical substances such as agents countering re-stenosis at the implantation site of the stent are a case in point.

An active principle may be loaded onto the implantable devices by means of a polymer applied/coated on the surface of the implantable device. The polymer thus acts as a reservoir or carrier of the active principle(s), which is (are) released from the device at the implantation site over a determined period of time and/or at a determined release rate.

A number of reasons have so far suggested the use of polymeric constituents behaving as carriers possibly adapted to modulate the release of the drug/drugs from e.g. a coronary stent.

The carrier function has to satisfy several requirements: being generally a coating on the stent surface, the polymer needs to adhere tightly to the platform and to follow all its movements; both these aspects require very good plasticity and adhesiveness, without elastic or sticky behavior of the polymer. Besides, the coating has not to compromise any mechanical property of the bare metal stent in all its functions, so as to say during expansion, recoil, detachment from the balloon, etc.

Moreover, polymers are generally well resistant to mechanical stresses likely to arise during the introduction into the human body e.g., in the case of stents, stresses due to a tortuous delivery path, calcified lesions, multiple stenting, etc.

Another noteworthy characteristic of polymers used as carriers of active principles (e.g. for coating drug eluting implantable prostheses) is the compatibility with the active principles associated therewith (mainly due to chemical inertia towards the active principles) without impairment of their carrier features.

Journal of Interventional Cardiology, Vol. 16, No. 6, 2003 (A. L. Bartorelli *et al.)* discloses stents provided on its outer surface with reservoirs having loaded within said reservoirs a drug, such as tacrolimus. This specific design allows the loading of the active agent in the surface of the stent without using a polymeric coating.

### OBJECT AND SUMMARY OF THE INVENTION

While the use of drug eluting implantable prostheses such as drug eluting stents has gained recognized medical efficacy, some issues are now increasingly raised about the safety of polymeric materials used for conveying and releasing the active principles.

For instance, polymeric substances coated/applied on an implantable device may remain *in situ* for long periods of time and thus undesirably disrupt or modify the healing process of the implantation site. This effect can be aggravated by an incomplete drug release from the carrier.

Moreover, concern as to possible cytotoxic effects of such polymers is rising: synthetic polymers (and polyester polymers particularly) seem to be exposed to degradation within the cells leading to toxic effects.

The object of the present invention is to provide compositions for the release of active principle(s) from implant devices, which overcome the disadvantages related to the use of polymeric materials.

According to the invention, the above object is achieved thanks to a composition as called for in the claims that follow. The invention also relates to an implant device having such composition loaded thereon as well as a corresponding use of such composition.

The claims are an integral part of the disclosure of the invention as provided herein.

Preferred embodiments of the invention provide drug eluting compositions that are not polymeric in their nature (even though they may possibly include polymers as additional components) and are able i) to modify, either by increasing or decreasing, the release of drug(s) from the implantable prosthesis and ii) to avoid the biological long-term adverse effects related to their presence on the implantable prosthesis.

Preferred embodiments of the invention concern drug eluting compositions that essentially take the form of pharmaceutical excipients to be combined with active principle(s).

Combination may occur in different ways, including but not limited to:
- admixing the active principle(s) and the excipient(s) when these are both in powder form;
- solubilising/suspending the active principle(s) and the excipient(s) in a co-solvent/vehicle to be possibly dispensed with;
- solubilising/suspending one of the active principle(s) or the excipient(s) into the other, when this latter is in liquid form.

A typical result to be obtained by combination is a matrix comprised of the excipient(s) loaded with the active principle(s) or vice-versa. Combination may also occur in the form of a layered structure with alternated layers, wherein each layer may be:
- an active principle layer;
- an excipient layer;
- a layer in the form of a matrix as described previously.

It will be appreciated that excipients included in the compositions as described herein can be typically classified, from a chemical point of view, as esters, waxes, fatty acids and esters of fatty acids, wherein the fatty acid can be saturated or unsaturated, hydrogenated or not, with short or long carbon chains.

Esters of natural compounds (e.g. glycerides and esters of sugars and vitamins) have been commonly used as vehicle/carrier materials for oral, parenteral and/or topical drug delivery and are well tolerated by the human body. For example, fatty acids glycerides are commonly present in emulsions for parenteral nutrition and particulate lipid matrices in the form of lipid pellets have been known for long for peroral administration and in cosmetics (liposomes).

Certain compositions described herein include at least one active principle to be delivered at the implantation site of an implant device and at least one excipient combined with said at least one active principle, said at least one excipient being in the form of a fatty acid ester of poly-alcohols, sugars or vitamins having a molecular weight not exceeding 30,000 (thirty thousand) Dalton. In another preferred embodiment, said at least one excipient is polyvinylpyrrolidone or polyethylenglycol having a molecular weight not exceeding 17,000 (seventeen thousand) Dalton.

Even without wishing to be tied to any specific theory in that respect, the inventors have reason to believe that such excipients having such molecular weight values (which approximately defines an upper threshold for the excipient(s) to be soluble in human tissues and/or being excreted by kidneys) reliably ensure that the excipient(s) as described herein is/are completely released from the implantable prosthesis as is the case of the drug, in a time period close or identical to that of the complete drug release, so that, at the end of the elution process, the implantable prosthesis returns to be a bare (typically metal) implantable device, free of any residues.

Presently preferred embodiments of the composition described herein provide for the at least one excipient to include esterified fatty acid, wherein the fatty acid may be selected among fatty acids with long chains between 15 and 22 carbon atoms or short chains up to 11 carbon atoms, saturated fatty acids and/or mono-unsaturated fatty acids.

Presently preferred embodiments of the composition described herein provide for the at least one fatty acid ester of glycerol being a mono- or di- or triglyceride or a mixture of them. The at least one fatty acid ester of glycerol may be glyceryl behenate, glyceryl monooleate and/or caprylic capric triglyceride.

The at least one excipient may include ethyl oleate, poliglycerol ester and/or at least one fatty acid ester of vitamins, such as ascorbyl palmitate.

Advantageously, the composition further includes pharmaceutically acceptable excipients selected from antioxydants, preservatives, solubilizers and stabilizers.

The at least one excipient may include polyvinylpyrrolidone (PVP).

The at least one active principle may be selected out of Tacrolimus, Paclitaxel, Sirolimus, Dexamethasone, Estradiol, Cilostazol, Thalidomide, their analogues, derivatives and groups of compounds. The at least one active principle may also be selected out of peptides, oligonucleotides or antibodies.

The relative amount of the at least one active principle present in the composition as described herein is between 1 and 99% by weight of said composition, and is mainly determined by the pharmacological potency/efficacy of the active principle itself.

In cases where the at least one active principle has an IC50 (50% inhibitory concentration) in the nanomolar order (like for example Paclitaxel) it is preferred that the at least one active principle is between 1 and 35%, further preferably between 3 and 33% by weight of said composition. Particularly preferred ranges are between 3.5 and 10%, and between 3.5 and 7% by weight of said composition. In other preferred embodiments, the at least one active principle is between 14 and 33% by weight of said composition.

In cases where the at least one active principle has an IC50 (50% inhibitory concentration) in the range between nanomolar and micromolar (like for example Tacrolimus) it is preferred that the at least one active principle is between 60 and 90%, preferably between 65 and 80% by weight of said composition. Also preferred is the range between 1 and 35%.

### BRIEF DESCRIPTION OF THE ANNEXED DRAWINGS

Further features and advantages of the invention will become apparent from the detailed description which follows and which is given purely by way of non limiting examples with reference to the annexed figures of drawings. These drawings are essentially in the form of diagrams showing exemplary delivery profiles of various active agents that may be achieved using compositions as described herein. Specifically:
- Figure 1 is representative of exemplary release profiles of Tacrolimus alone, Tacrolimus:PVP at weight ratios 60:40 and 80:20;
- Figure 2 is representative of exemplary release profiles of Tacrolimus/Ascorbyl Palmitate at weight ratios 90:10 and 80:20;
- Figure 3 is representative of exemplary release profiles of Tacrolimus alone, Tacrolimus:PVP at a weight ratio 80:20 and Tacrolimus:Ascorbyl Palmitate at a weight ratio 80:20;
- Figure 4 is representative of exemplary release profiles of Paclitaxel alone and Paclitaxel:Compritol at a weight ratio 7:93;
- Figure 5 is representative of exemplary release profiles of Estradiol Valerate:Compritol at a weight ratio 65:35 and Dexamethasone acetate:Compritol at a weight ratio 65:35.

### DETAILED DESCRIPTION OF EXEMPLARY EMBODIMENTS

While the exemplary embodiments detailed in the following primarily relate to the use the compositions described herein in connection with angioplasty stents, it will be understood that the scope of this disclosure is in no way limited to that prospected use, since the compositions described herein can be used in connection with any type of implantable device, such as e.g. prosthetic valves.

Additionally, while the experimental data provided in the following relate to the use of the compositions described herein in connection with certain active principles (mainly of the lypophilic type), the scope of this disclosure is in no way limited to these specific active principles, in that the compositions described herein exhibit an excellent degree of compatibility with other types of active principles, such as hydrophilic compounds.

The compositions as described herein are primarily intended to modulate the release process and thus the bio-availability of the active principle(s) - i. e. the "drug(s)" - loaded onto the implant device.

Additionally, the compositions as described herein are completely released from the implantable prosthesis as is the case of the drug, in a time period close or identical to that of the complete drug release. Consequently, at the end of the elution process the implantable prosthesis returns to be a bare (typically metal) implantable device, free of any residues.

The compositions as described herein do not require *in situ* degradation steps in order to be removed from the implantable prosthesis to be then excreted and/or metabolized by the human body: as used herein, the expression *"in* situ degradation" is intended to indicate, in general, any modification before detachment or dissolution and removal from the stent surface.

Preferred compositions as described herein are comprised of natural compounds that are highly biocompatible, if not constituents of human tissues. Such compounds will normally be excreted by the human body or will enter the normal metabolic cycles of the organism. As these compositions leave the implanted prosthesis, typically together with the drug, they can also modify the "microenvironment" around the prosthesis and actively modulate drug elution and diffusion, influencing metabolic processes or acting as vehicles.

The compositions as described herein thus strongly differ from the polymers commonly used in drug eluting implantable prosthesis, in that those polymers are an inert physical barrier unable to migrate with the active principle or e.g. to increase its elution. Those polymers can only passively slow down the rate of drug release.

The compositions as described herein may exhibit significant auxiliary functions because of their chemical-physical nature; they may, in fact, be antioxidant, antimicrobial, stabilizing agents, permeation-enhancers, etc. Preferred substances are those that act through natural/physiological mechanisms, such as vitamins and food lipids. For the reasons explained above, the compositions considered herein can protect the drug both when present on the medical device and in the physiological environment, increasing drug stability and modifying its therapeutic window.

As it will be apparent from the experiments described in the following, the compositions described herein achieve the following results:
- They are able to speed up or slow down the dissolution rate of the drug; in particular, they are able to change the release rate of the drug by using different percentages of excipient(s);
- Typically, they be completely released from the implantable prosthesis surface and are, in fact, degraded, excreted and/or metabolized by the human body without leaving any residues.

These results were confirmed by *in* vitro experiments as well as in several *in vivo* tests. The in vivo experiments were carried out on rabbits and pigs that were implanted with drug eluting stents either orthotopically or in iliac arteries. The ability of such compositions to modulate the release of the drug and the extremely high biocompatibility at different follow-up time points were demonstrated.

As indicated, the compositions as described herein can be classified, from a chemical point of view, as esters, waxes, fatty acids and esters of fatty acids, wherein the fatty acid can be saturated or unsaturated, hydrogenated or not, with short or long carbon chains. In particular, esters of glycerol, polyalcohols, sugars and complex alcohols are preferred, particularly preferred are fatty acids esters of vitamins.

Esters of natural compounds are, then, compatible with both poorly and freely water-soluble drugs, are able to improve the chemical and physical stability and to modify the *in vivo* release and bioavailability of the associated drug at the site of action.

The use of esters of natural compounds in combination with small amounts of other excipients, like for example solubilizers, stabilizers, antioxidants and antimicrobials, allows to produce solid matrices, whose solid state permits prolonged drug release and protects incorporated active ingredients against chemical degradation.

Other natural or synthetic excipients may be included in the compositions as described herein, provided that such other (additional) excipients leave the implantable prosthesis without being subject to in situ degradation, are able to modulate the release rate of the drug and its bioavailability and to increase the physical stability of the drug. Preferred examples of synthetic excipients for use as such additional excipients are polyvynylpirrolidone and polyethylenglycol.

The compositions as described herein can be lipophilic, hydrophilic or amphyphilic and their selection shall depend on the drug characteristics, on the release modulation and tissues diffusion requirements. Lypophilic compositions are preferred at present, as they are compatible with drugs normally used on drug eluting stents.

Particularly preferred are those compositions that change their physical state at a certain pH or temperature, especially if the pH or temperature are in the physiological range.

The compositions as described herein currently exhibit good workability, which facilitates loading onto the implantable prosthesis, and resist manufacturing steps of implantable prostheses.

Thanks to their different physical properties, the compositions as described herein can be stratified e.g. on drug eluting stents (DES). This may be useful in the preparation of DES with layers containing different drugs or a single drug with different kinetic profiles.

The compositions as described herein may be reduced in powder form, dissolved, suspended, made in paste form, or in particles of different sizes (micro or nano-spheres) for being loaded onto the implantable prosthesis.

The compositions as described herein may be associated essentially as excipients with one or more drugs, before or after being loaded onto the implantable prosthesis. Drug/excipient ratio will be adjusted to obtain the correct drug release profile or to produce the desired effects on drug stability or drug physical state. The drug/excipient formulations may, then, be solved, solvated and/or heat-treated to fix them onto the implantable prosthesis.

Drugs loadable onto an implantable device may be selected among, but not limited to, the following classes: anti-inflammatory, anti-proliferative, wound pro-healing, tyrosine kinase inhibitors, immunosuppressors and anti-tumor agents. Particular attention is given to drugs such as Tacrolimus, Paclitaxel, Sirolimus, Dexamethasone, corticosteroids, Estradiol, Cilostazol, Thalidomide and their analogues and derivatives.

Again, it is stressed that the scope of this disclosure is in no way limited to these specific active principles, in that the compositions described herein exhibit an excellent degree of compatibility with other types of active principles, such as e.g. hydrophilic compounds.

### EXAMPLES

Stents have been loaded with different formulations containing different drugs. Dissolution experiments have been performed, measuring the drug amount released from the sent during time.

The stents had on their outer surface reservoirs, in the form of grooves, able to contain the drugs. At the end of the dissolution experiments the stent reservoirs were completely empty. This is in contrast to drugs released from stents by means of polymers according to the prior art, where at the end of the dissolution experiment, a part of the polymer would still be within the reservoirs.

### MATERIALS AND METHODS

### Stent description

Stainless steel (Janus Carbostent® as produced by Sorin Biomedica Cardio S.p.A. of Saluggia - Italy) or cobalt chromium stents (produced as test run at Sorin) were employed, both types of stents having groove-like reservoirs for active principles laser cut in the outer surface.

### Drug release formulation composition

The substances employed as drugs were:
- Tacrolimus or FK 506 (drug),
- Paclitaxel (drug),
- Estradiol valerate (drug),
- Dexamethasone acetate (drug).

The excipients used for the compositions in association with the drugs were:
- glyceryl behenate(Compritol 888 ATO),
- Polyvinyl pyrrolidone (PVP) 17K,
- Ascorbyl Palmitate.

The stents have been loaded with the following formulations:
- Tacrolimus;
- Tacrolimus:Ascorbyl Palmitate in a weight ratio 80:20;
- Tacrolimus:Ascorbyl Palmitate in a weight ratio 60:40;
- Tacrolimus:PVP in a weight ratio 90:10;
- Tacrolimus:PVP in a weight ratio 80:20;
- Paclitaxel;
- Paclitaxel:Compritol in a weight ratio 7:93;
- Estradiol Valerate:Compritol in a weight ratio 65:35;
- Dexamethasone Acetate:Compritol in a weight ratio 65:35.

### Preparation of the different formulations drug: excipient (s)

The Tacrolimus:PVP and Tacrolimus:Ascorbyl Palmitate formulations are obtained by physically mixing the components in the desired amounts.

The formulations involving Compritol are prepared as follows. The drug is incorporated into the formulation by means of the well-known co-melting technique. In particular, the excipient is first melted in a thermostatic bath at a proper temperature. The active drug is then added stepwise to the molten mixture under constant magnetic or mechanical stirring.

The formulation can also be prepared using the solvent stripping technique. The drug and the excipient(s) are first co-suspended or co-solubilized at a suitable temperature in a proper solvent, which is subsequently stripped at proper conditions (e.g. under vacuum at a suitable temperature or at controlled atmosphere). In this first step additional excipients, such as phospholipids, solubilizers, stabilizer and antimicrobial agents could be added.

When employing the solvent-stripping technique, the solvent is preferably selected from the group consisting of water, ethanol, butanol, propanol, benzylalcohol, isopropanol, dioxane, methylenechloride or a mixture thereof.

The formulation may be optionally stabilized at proper selected conditions of storage (temperature, humidity, light) in order to obtain a solid formulation.

The physical properties of the formulation before and after the loading onto the stent can be optimized using different techniques (e.g. milling, spray-drying, cryogenic milling, fluid bed, cryogenic bath, electric field, supercritic fluid spray).

### In vitro drug dissolution experiments

Stents are kept in the correct volume of dissolution medium (slightly modified phosphate buffer at pH=7.4) at 37°C in constant agitation.

At each time point a sample of the dissolution medium is picked up and analysed for the drug concentration. The amount of dissolution medium picked up is replaced with the same amount of fresh medium.

Samples are analysed by HPLC (High Performance Liquid Chromatography) and the total amount of released drug is calculated at each time point.

Experiments are prolonged until the complete release of the formulation from the stent is achieved (≥ 80% loaded drug dose). At the end of the experiment, the stent is observed by means of a microscope to verify whether the reservoirs are empty, and, then, the stent is dried and weighted to confirm that nothing remains on the stent itself.

In a first set of experiments, the effect of different percentages of the same excipient with the same drug was studied.

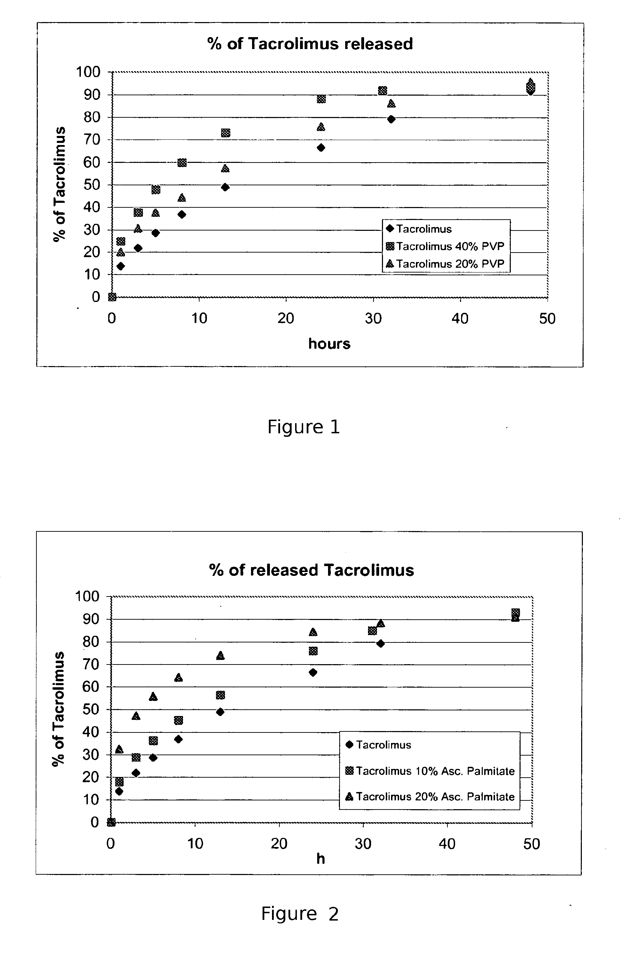

The release profiles of Tacrolimus alone, Tacrolimus:PVP 80:20 and Tacrolimus:PVP 60:40 are shown in figure 1. In figure 2 the same experiment is repeated with Tacrolimus:Ascorbyl Palmitate 90:10 and 80:20.

In both experiments Tacrolimus release rate is increased as the percentage of excipient increases. Total release of the different formulations is achieved at the end of the experiment.

In a second set of experiments two excipients are compared, at the same percentage, in formulations with the same drug.

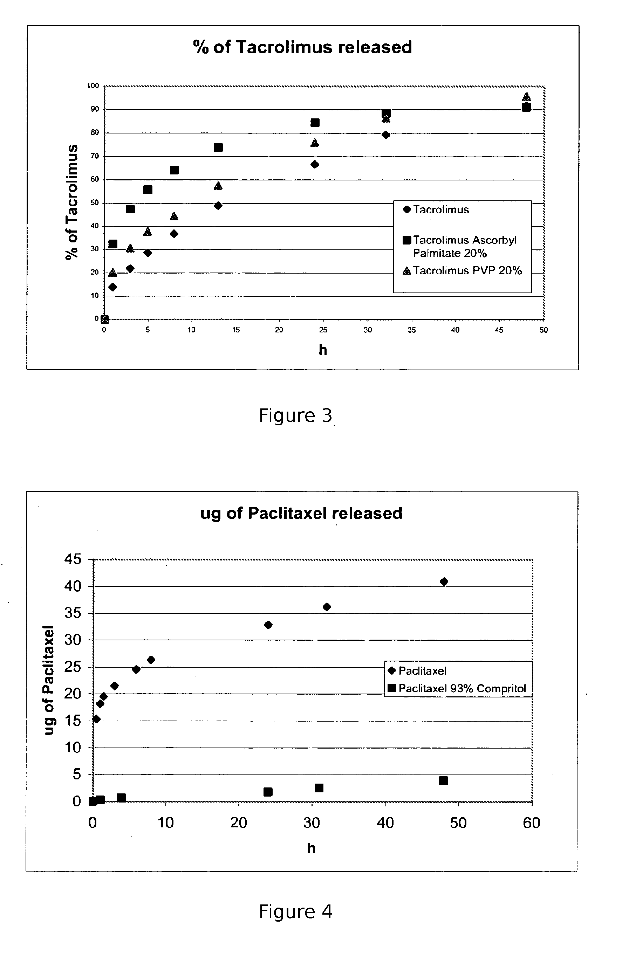

In figure 3 release profiles of Tacrolimus alone, Tacrolimus:PVP 80:20 and Tacrolimus:Ascorbyl Palmitate 80:20 are reported.

The drug dissolution rate is increased in a different manner by the two different excipients. Total release of the different formulations is achieved at the end of the experiment.

Excipients can be used to reduce drug dissolution rate not only to speed it up.

In figure 4 dissolution profiles of Paclitaxel alone and Paclitaxel:Compritol 7:93 are compared.

The presence of the excipient strongly reduces drug dissolution rate. Total release of the different formulations is achieved at the end of the experiment.

The same excipient can differently modulate the dissolution behaviour of different drugs.

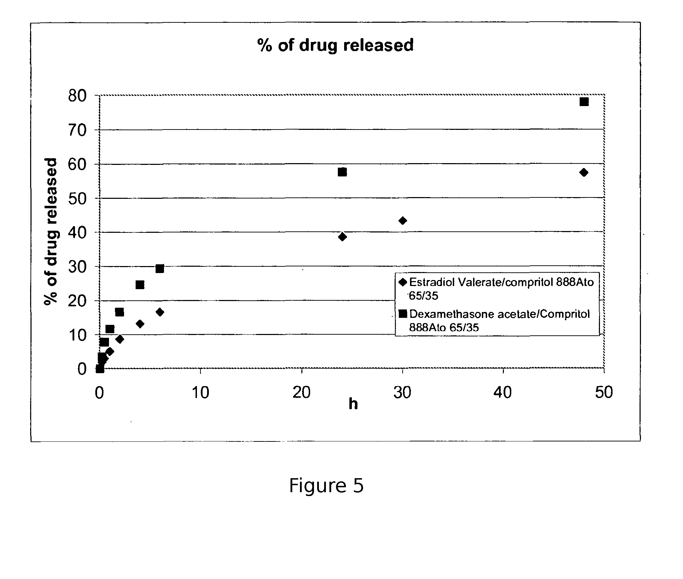

In figure 5, Estradiol Valerate:Compritol 65:35 and Dexamethasone acetate:Compritol 65:35 are compared.

The two formulations contain the same percentage of the same excipient but the drug dissolution rate is different for the two different drugs. Total release of the different formulations is achieved at the end of the experiment.

## Claims

1. A stent provided on its outer surface with reservoirs in the form of grooves having loaded within said reservoirs a composition including:
- at least one active principle to be delivered at the implantation site of the implant device, and
- at least one excipient combined with said at least one active principle, said at least one excipient being selected among fatty acids esters of polyalcohols, sugars or vitamins having a molecular weight not exceeding 30,000 Dalton.

2. The stent of claim 1, wherein said at least one excipient is soluble in human tissues.

3. The stent of claim 1, wherein said at least one excipient includes at least one fatty acid ester of glycerol.

4. The stent of claim 3, wherein said at least one fatty acid ester of glycerol is a mono- or di- or tri-glyceride or a mixture of them.

5. The stent of either of claims 3 or 4, wherein said at least one fatty acid ester of glycerol is glyceryl behenate.

6. The stent of either of claims 3 or 4, wherein said at least one fatty acid ester of glycerol is glyceryl monooleate.

7. The stent of either of claims 3 or 4, wherein said at least one fatty acid ester of glycerol is caprylic/capric triglyceride.

8. The stent of any of claims 3 to 7, wherein said at least one excipient includes ethyl oleate.

9. The stent of any of claims 3 to 8, wherein said at least one excipient includes poliglycerol ester.

10. The stent of claim 1, wherein said at least one fatty acid ester of vitamins is ascorbyl palmitate.

11. The stent of any of claims 1 to 10, wherein the esterified fatty acid is selected among fatty acids with long chains between 15 and 22 carbon atoms or short chains up to 11 carbon atoms.

12. The stent of any of claims 1 to 11, wherein the esterified fatty acid is selected among saturated fatty acids.

13. The stent of any of claims 1 to 12, wherein the esterified fatty acid is selected among mono-unsaturated fatty acids.

14. The stent of any of the preceding claims wherein the composition further includes pharmaceutically acceptable excipients selected from antioxydants, preservatives, solubilizers and stabilizers.

15. A stent provided on its outer surface with reservoirs in the form of grooves having loaded within said reservoirs a composition including:
- at least one active principle to be delivered at the implantation site of the implant device, and
- at least one excipient combined with said at least one active principle, said at least one excipient being selected between polyvinylpyrrolidone (PVP) and polyethylenglycol having a molecular weight not exceeding 17,000 Dalton.

16. The stent of any of the preceding claims, wherein said at least one active principle is selected out of Tacrolimus, Paclitaxel, Sirolimus, Dexamethasone, Estradiol, Cilostazol, Thalidomide, their analogues, derivatives and groups of compounds.

17. The stent of any of the preceding claims, wherein said at least one active principle is selected out of peptides, oligonucleotides or antibodies.

18. The stent of any of the preceding claims, wherein said at least one active principle is between 1 and 99% by weight of said composition.

19. The stent of any of the preceding claims, wherein, when said active principle has an IC50 of the order of nanomolar, said at least one active principle is between 1 and 35% by weight of said composition.

20. The stent of claim 19, wherein said at least one active principle is between 3 and 33% by weight of said composition.

21. The stent of claim 20, wherein said at least one active principle is between 3.5 and 10% by weight of said composition.

22. The stent of claim 21, wherein said at least one active principle is between 3.5 and 7% by weight of said composition.

23. The stent of claim 19, wherein said at least one active principle is between 14 and 33% by weight of said composition.

24. The stent of claim 19, wherein, when said active principle has an IC50 in the range between nanomolar and micromolar, said at least one active principle is between 60 and 90% by weight of said composition.

25. The stent of claim 24, wherein said least one active principle is between 65 and 80% by weight of said composition.

26. The stent of claim 18, wherein, when said active principle has an IC50 in the range between nanomolar and micromolar, said at least one active principle is between 1% and 35% by weight of said composition.

## Patentansprüche

1. Stent, an dessen Außenseite rillenförmige Reservoirs vorgesehen sind, die mit einem Gemisch befüllt sind, welches umfasst
- wenigstens einen Wirkstoff, der an die Implantationsstelle der Implantationsvorrichtung abzugeben ist, und
- wenigstens einen Hilfsstoff, der mit dem wenigstens einen Wirkstoff kombiniert ist, wobei der wenigstens eine Hilfsstoff ausgewählt ist aus Fettsäureestern von Polyalkoholen, Zuckern oder Vitaminen, die ein molekulares Gewicht aufweisen, welches 30.000 Dalton nicht überschreitet.

2. Stent nach Anspruch 1, **dadurch gekennzeichnet, dass** der wenigstens eine Hilfsstoff in menschlichem Gewebe lösbar ist.

3. Stent nach Anspruch 1, **dadurch gekennzeichnet, dass** der wenigstens eine Hilfsstoff wenigstens einen Fettsäureester des Glycerols umfasst.

4. Stent nach Anspruch 3, **dadurch gekennzeichnet**, das der wenigstens eine Fettsäureester des Glycerols ein Mono- oder Di- oder Tri-Glycerid oder eine Mischung dieser ist.

5. Stent nach Anspruch 3 oder 4, **dadurch gekennzeichnet, dass** der wenigstens eine Fettsäureester des Glycerols Glycerylbehenat ist.

6. Stent nach Anspruch 3 oder 4, **dadurch gekennzeichnet, dass** der wenigstens eine Fettsäureester des Glycerols Glycerylmonooleat ist.

7. Stent nach Anspruch 3 oder 4, **dadurch gekennzeichnet, dass** der wenigstens eine Fettsäureester des Glycerols Capryl-/Caprin-Triglycerid ist.

8. Stent nach einem der Ansprüche 3 bis 7, **dadurch gekennzeichnet, dass** der wenigstens eine Hilfsstoff Ethyloleat umfasst.

9. Stent nach einem der Ansprüche 3 bis 8, **dadurch gekennzeichnet, dass** der wenigstens eine Hilfsstoff Polyglycerolester umfasst.

10. Stent nach Anspruch 1, **dadurch gekennzeichnet, dass** der wenigstens eine Fettsäureester von Vitaminen Ascorbylpalmitat ist.

11. Stent nach einem der Ansprüche 1 bis 10, **dadurch gekennzeichnet, dass** die veresterte Fettsäure ausgesucht ist aus Fettsäuren mit langen Ketten zwischen 15 und 22 Kohlenstoffatomen oder kurzen Ketten bis zu 11 Kohlenstoffatomen.

12. Stent nach einem der Ansprüche 1 bis 11, **dadurch gekennzeichnet, dass** die veresterte Fettsäure ausgesucht ist aus gesättigten Fettsäuren.

13. Stent nach einem der Ansprüche 1 bis 12, **dadurch gekennzeichnet, dass** die veresterte Fettsäure ausgesucht ist aus einfach ungesättigten Fettsäuren.

14. Stent nach einem der vorhergehenden Ansprüche, **dadurch gekennzeichnet, dass** das Gemisch weiterhin pharmazeutisch akzeptable Hilfsstoffe, ausgesucht aus Antioxidantien, Konservierungsmitteln, Lösungsvermittlern und Stabilisierern, umfasst.

15. Stent, an dessen Außenseite rillenförmige Reservoirs vorgesehen sind, die mit einem Gemisch befüllt sind, welches umfasst
- wenigstens einen Wirkstoff, der an die Implantationsstelle der Implantationsvorrichtung abzugeben ist und
- wenigstens einen Hilfsstoff, der mit dem wenigstens einen Wirkstoff kombiniert ist, wobei der wenigstens eine Hilfsstoff ausgewählt ist aus Polyvinylpyrrolidon (PVP) und Polyethylenglycol, die ein molekulares Gewicht aufweisen, welches 17.000 Dalton nicht überschreitet.

16. Stent nach einem der vorhergehenden Ansprüche, **dadurch gekennzeichnet, dass** der wenigstens eine Wirkstoff ausgewählt ist aus Tacrolimus, Paclitaxel, Sirolimus, Dexamethason, Estradiol, Cilostazol, Thalidomid, deren Analoga, Derivaten und Gruppen von Bestandteilen.

17. Stent nach einem der vorhergehenden Ansprüche, **dadurch gekennzeichnet, dass** der wenigstens eine Wirkstoff ausgewählt ist aus Peptiden, Oligonukleotiden oder Antikörpern.

18. Stent nach einem der vorhergehenden Ansprüche, **dadurch gekennzeichnet, dass** der wenigstens eine Wirkstoff zwischen 1 und 99 Gew.-% des Gemischs liegt.

19. Stent nach einem der vorhergehenden Ansprüche, **dadurch gekennzeichnet, dass**, wenn der Wirkstoff einen IC50 im nanomolaren Bereich aufweist, der wenigstens eine Wirkstoff zwischen 1 und 35 Gew.-% des Gemischs liegt.

20. Stent nach Anspruch 19, **dadurch gekennzeichnet, dass** der wenigstens eine Wirkstoff zwischen 3 und 33 Gew.-% des Gemischs liegt.

21. Stent nach Anspruch 20, **dadurch gekennzeichnet, dass** der wenigstens eine Wirkstoff zwischen 3,5 und 10 Gew.-% des Gemischs liegt.

22. Stent nach Anspruch 21, **dadurch gekennzeichnet, dass** der wenigstens eine Wirkstoff zwischen 3,5 und 7 Gew.-% des Gemischs liegt.

23. Stent nach Anspruch 19, **dadurch gekennzeichnet, dass** der wenigstens eine Wirkstoff zwischen 14 und 33 Gew.-% des Gemischs liegt.

24. Stent nach Anspruch 19, **dadurch gekennzeichnet, dass**, wenn der Wirkstoff ein IC50 im Bereich zwischen nanomolar und mikromolar aufweist, der wenigstens eine Wirkstoff zwischen 60 und 90 Gew.-% des Gemischs liegt.

25. Stent nach Anspruch 24, **dadurch gekennzeichnet, dass** der wenigstens eine Wirkstoff zwischen 65 und 80 Gew.-% des Gemischs liegt.

26. Stent nach Anspruch 18, **dadurch gekennzeichnet, dass**, wenn der Wirkstoff ein IC50 im Bereich zwischen nanomolar und mikromolar aufweist, der wenigstens eine Wirkstoff zwischen 1 und 35 Gew.-% des Gemischs liegt.

## Revendications

1. Stent doté de réservoirs sous forme de rainures sur sa surface externe, où lesdits réservoirs sont chargés avec une composition comprenant :
- au moins un principe actif devant être délivré au niveau du site d'implantation du dispositif d'implant, et
- au moins un excipient combiné avec ledit au moins un principe actif, ledit au moins un excipient étant sélectionné parmi des esters d'acide gras de polyalcools, de sucres ou de vitamines ayant un poids moléculaire n'excédant pas 30 000 Daltons.

2. Stent selon la revendication 1, dans lequel ledit au moins un excipient est soluble dans des tissus humains.

3. Stent selon la revendication 1, dans lequel ledit au moins un excipient comprend au moins un ester d'acide gras de glycérol.

4. Stent selon la revendication 3, dans lequel ledit au moins un ester d'acide gras de glycérol est un mono- ou un di- ou un triglycéride ou un mélange de ceux-ci.

5. Stent selon l'une quelconque des revendications 3 ou 4, dans lequel ledit au moins un ester d'acide gras de glycérol est le béhénate de glycéryle.

6. Stent selon l'une quelconque des revendications 3 ou 4, dans lequel ledit au moins un ester d'acide gras de glycérol est le monooléate de glycéryle.

7. Stent selon l'une quelconque des revendications 3 ou 4, dans lequel ledit au moins un ester d'acide gras de glycérol est un triglycéride caprylique/caprique.

8. Stent selon l'une quelconque des revendications 3 à 7, dans lequel ledit au moins un excipient comprend de l'oléate d'éthyle.

9. Stent selon l'une quelconque des revendications 3 à 8, dans lequel ledit au moins un excipient comprend un ester de polyglycérol.

10. Stent selon la revendication 1, dans lequel ledit au moins un ester d'acide gras de vitamines est le palmitate d'ascorbyle.

11. Stent selon l'une quelconque des revendications 1 à 10, dans lequel l'acide gras estérifié est sélectionné parmi des acides gras à longues chaînes comportant entre 15 et 22 atomes de carbone ou à courtes chaînes comportant jusqu'à 11 atomes de carbone.

12. Stent selon l'une quelconque des revendications 1 à 11, dans lequel l'acide gras estérifié est sélectionné parmi des acides gras saturés.

13. Stent selon l'une quelconque des revendications 1 à 12, dans lequel l'acide gras estérifié est sélectionné parmi des acides gras mono-insaturés.

14. Stent selon l'une quelconque des revendications précédentes, dans lequel la composition comprend en outre des excipients pharmaceutiquement acceptables sélectionnés parmi des antioxydants, des conservateurs, des solubilisants et des stabilisateurs.

15. Stent doté de réservoirs sous forme de rainures sur sa surface externe, où lesdits réservoirs sont chargés avec une composition comprenant :
- au moins un principe actif devant être délivré au niveau du site d'implantation du dispositif d'implant, et
- au moins un excipient combiné avec ledit au moins un principe actif, ledit au moins un excipient étant sélectionné parmi la polyvinylpyrrolidone (PVP) et le polyéthylène glycol ayant un poids moléculaire n'excédant pas 17 000 Daltons.

16. Stent selon l'une quelconque des revendications précédentes, dans lequel ledit au moins un principe actif est sélectionné parmi le tacrolimus, le paclitaxel, le sirolimus, la déxaméthasone, l'oestradiol, le cilostazol, le thalidomide, leurs analogues, dérivés et groupes de composés.

17. Stent selon l'une quelconque des revendications précédentes, dans lequel ledit au moins un principe actif est sélectionné parmi des peptides, des oligonucléotides ou des anticorps.

18. Stent selon l'une quelconque des revendications précédentes, dans lequel ledit au moins un principe actif est compris entre 1 et 99 % en poids de ladite composition.

19. Stent selon l'une quelconque des revendications précédentes, dans lequel ledit principe actif possède une IC50 de l'ordre du nanomolaire, ledit au moins un principe actif étant compris entre 1 et 35 % en poids de ladite composition.

20. Stent selon la revendication 19, dans lequel ledit au moins un principe actif est compris entre 3 et 33 % en poids de ladite composition.

21. Stent selon la revendication 20, dans lequel ledit au moins un principe actif est compris entre 3,5 et 10 % en poids de ladite composition.

22. Stent selon la revendication 21, dans lequel ledit au moins un principe actif est compris entre 3,5 et 7 % en poids de ladite composition.

23. Stent selon la revendication 19, dans lequel ledit au moins un principe actif est compris entre 14 et 33 % en poids de ladite composition.

24. Stent selon la revendication 19, dans lequel, lorsque ledit principe actif possède une IC50 dans la plage nanomolaire à micromolaire, ledit au moins un principe actif est compris entre 60 et 90 % en poids de ladite composition.

25. Stent selon la revendication 24, dans lequel ledit au moins un principe actif est compris entre 65 et 80 % en poids de ladite composition.

26. Stent selon la revendication 18, dans lequel, lorsque ledit principe actif possède une IC50 dans la plage nanomolaire à micromolaire, ledit au moins un principe actif est compris entre 1 % et 35 % en poids de ladite composition.
